# EUROPEAN PATENT SPECIFICATION

(11) **EP 4 163 357 B1**
(45) Date of publication and mention of the grant of the patent: **06.08.2025**
(21) Application number: 20939354.5
(22) Date of filing: 03.06.2020
(51) Int. Cl.: C12M 1/00, G01N 21/64, G01N 21/78, C12Q 1/00, G01N 1/31, G01N 35/10

(54) **NUCLEIC ACID ANALYZER**
NUKLEINSÄUREANALYSEGERÄT
ANALYSEUR D'ACIDE NUCLÉIQUE

(43) Date of publication of application: 12.04.2023
(73) Proprietor: HITACHI HIGH-TECH CORPORATION, Tokyo 105-6409 (JP)
(72) Inventor: YAMASHITA Tatsuya, Tokyo 105-6409 (JP); SHOJI Tomohiro, Tokyo 105-6409 (JP); ONUKI Kazutoshi, Tokyo 105-6409 (JP)
(74) Representative: Strehl & Partner mbB
(86) International application number: PCT/JP2020/022025
(87) International publication number: WO 2021/245859

(56) References cited:
- EP-A1- 0 969 083
- WO-A1-2007/058077
- WO-A1-2015/098784
- WO-A1-2020/100659
- JP-A- 2004 028 589
- JP-A- 2009 532 031
- JP-A- 2015 065 956
- JP-A- 2019 187 256
- JP-A- 2020 020 601
- JP-A- H07 274 935
- US-A1- 2004 121 356
- US-A1- 2004 146 874
- US-A1- 2005 266 582
- US-A1- 2010 111 768

## Description

### Technical Field

The present invention relates to a nucleic acid analyzer.

### Background Art

A nucleic acid analyzer is known as a device for analyzing base sequences of a deoxyribonucleic acid (DNA). The nucleic acid analyzer is a device for analyzing the base sequences of DNA by denaturing DNA fragments into a single strand, using the single strand as a model, extending a nucleic acid attached with a fluorescent label by one base each time, and sequentially capturing a fluorescent image. When analysis is performed, a substrate in which a flow path is provided in a plate made of a partially or entirely transparent material is prepared, and colonies containing a plurality of cloned DNA fragments denatured into a single strand are fixed in a reaction field provided in the flow path of the substrate. In order to enable identification of four types of nucleotides (adenine, cytosine, guanine, thymine) forming DNA for the colonies containing the plurality of DNA fragments, a reagent that fluorescently labels each base of DNA, a reagent that cleans the flow path, and the like are alternately sent. The base sequences of DNA can be sequentially analyzed by capturing, as a fluorescent image, a process in which the colonies are restored to contain the double-stranded DNA fragments.

In analysis of base sequences of DNA in the nucleic acid analyzer, first, for the colonies containing the plurality of DNA fragments fixed in the reaction field provided in the flow path of the substrate, a reagent required for each reaction process is selected from a plurality of types of reagents and is sent to the flow path of the substrate, and thus each base of the colonies containing the DNA fragments in the flow path is fluorescently modified. Further, the colonies containing the fluorescently modified DNA fragments are observed. The base sequences are analyzed as described above.

In such a nucleic acid analyzer, it is considered to increase an area of the reaction field by providing a plurality of flow paths on the substrate in order to improve throughput. A nucleic acid analyzer using a substrate having a plurality of flow paths is reported in PTL 1.

### Citation List

### Patent Literature

PTL 1: US8241573B2
Further, EP 0969083 A1, WO 2015/098784 A1 and US 2004/0121356 A1 disclose analyzers of the prior art from which the pre-characterising part of claim 1 starts out.

### Summary of Invention

### Technical Problem

PTL 1 describes a configuration of a nucleic acid analyzer that sends a reagent to a substrate having a plurality of flow paths. However, the nucleic acid analyzer in PTL 1 requires a branched flow path structure for connecting reagents with a plurality of substrate flow paths in order to introduce the reagents into the plurality of flow paths, and the substrate flow paths and the branched flow path are both required to be replaced with the reagents, and thus a reagent consumption amount increases.

In addition, when it is considered to use substrates of different sizes in order to cope with a plurality of types of throughput (for example, a substrate having a half number of flow paths is used in order to implement nucleic acid analysis at half throughput), a branched flow path that is not connected to the substrate is generated in the branched flow path structure. A branched flow path portion that is not connected to the substrate becomes a dead volume, and a remaining drug solution or bubbles cannot be replaced with the reagents, thereby causing contamination. Therefore, it is not desirable to use substrates of different sizes.

In view of the above problems, an object of the invention is to provide a nucleic acid analyzer capable of mounting a plurality of types of substrates having different numbers of flow paths while preventing an increase in reagent consumption amount due to a branched flow path structure.

### Solution to Problem

The above problem is solved by a nucleic acid analyzer as set forth in the appended claims.

### Advantageous Effects of Invention

According to the nucleic acid analyzer of the invention, by providing a branch point of the flow paths in the substrate, it is possible to minimize the number of introduction flow paths and eliminate an increase in reagent consumption amount due to an increase in the number of flow paths of the substrate. Further, by stabilizing the number of inlet portions of the substrate regardless of the number of flow paths on the substrate, it is possible to mount a plurality of types of substrates having different numbers of flow paths without generating a dead volume in the same device configuration.

### Brief Description of Drawings

[FIG. 1] FIG. 1 shows a configuration diagram of a nucleic acid analyzer 100 according to a first embodiment.
[FIG. 2] FIG. 2 shows a configuration diagram when a substrate 107 is mounted on the nucleic acid analyzer 100.
[FIG. 3] FIG. 3 shows a configuration diagram when a substrate 301 having a size different from that of the substrate 107 is mounted on the nucleic acid analyzer 100.
[FIG. 4] FIG. 4 shows a configuration diagram of the nucleic acid analyzer 100 according to a second embodiment.
[FIG. 5] FIG. 5 shows a configuration diagram of the nucleic acid analyzer 100 according to a third embodiment.
[FIG. 6] FIG. 6 shows a configuration diagram of a block 501.
[FIG. 7] FIG. 7 is a configuration diagram of the nucleic acid analyzer 100 according to a fourth embodiment.
[FIG. 8] FIG. 8 is a diagram showing a state in which the substrate 301 is used.
[FIG. 9] FIG. 9 is a configuration diagram of the substrates 107 and 301 used in the nucleic acid analyzer 100 according to a fifth embodiment.

### Description of Embodiments

### <First Embodiment>

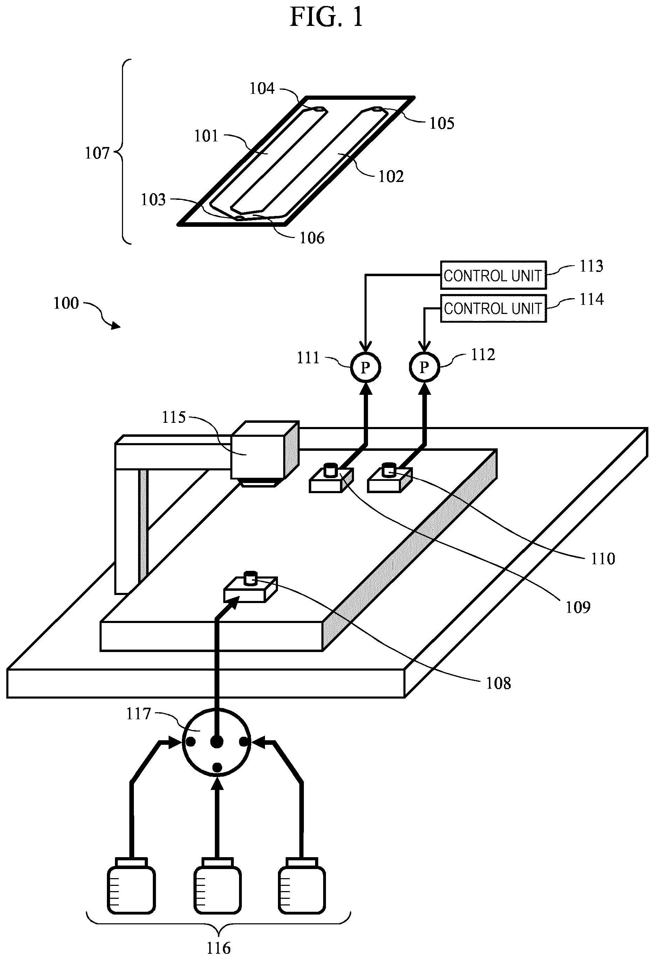

FIG. 1 shows a configuration diagram of a nucleic acid analyzer 100 according to a first embodiment of the invention. The nucleic acid analyzer 100 includes a substrate 107, an introduction flow path 108, discharge flow paths 109 and 110, reagent aspiration mechanisms 111 and 112, control units 113 and 114, an imaging mechanism 115, reagent containers 116, and a reagent selection mechanism 117. The substrate 107 can be attached to and detached from a device main body. Other components are provided on a device main body side.

The substrate 107 includes at least two flow paths 101 and 102, at least one inlet portion 103, at least two outlet portions 104 and 105, and at least one flow path branch point 106. The flow paths 101 and 102 are used to fix colonies containing DNA fragments and analyze base sequences of DNA. Each reagent is introduced into the substrate 107 from the inlet portion 103. The reagent is discharged from the outlet portions 104 and 105. The flow path branch point 106 is a location at which a flow path is branched into flow paths (a merging point of the flow paths). The flow path 101 connects between the inlet portion 103 and the outlet portion 104, and the flow path 102 connects between the inlet portion 103 and the outlet portion 105.

The introduction flow path 108 is connected to the inlet portion 103. The reagent is introduced into the substrate 107 via the introduction flow path 108 and the inlet portion 103. The discharge flow path 109 is connected to the outlet portion 104, and the discharge flow path 110 is connected to the outlet portion 105. The reagent aspiration mechanism 111 aspirates the reagent flowing through the discharge flow path 109, and the reagent aspiration mechanism 112 aspirates the reagent flowing through the discharge flow path 110. The control unit 113 controls the reagent aspiration mechanism 111, and the control unit 114 controls the reagent aspiration mechanism 112. The imaging mechanism 115 captures a fluorescent image of the colonies containing the DNA fragments. Reagents are contained in the reagent containers 116. The reagent selection mechanism 117 selects a reagent to be introduced into the substrate 107 by selectively connecting to any one of the reagent containers 116.

In FIG. 1, one inlet portion 103 of the substrate 107 and one introduction flow path 108 are provided, and two or more inlet portions 103 and two or more introduction flow paths 108 may be provided as long as the number of the inlet portions 103 and the number of the introduction flow paths 108 are both smaller than the number of the outlet portions 104 and 105. In FIG. 1, two outlet portions 104 and 105 and two discharge flow paths 109 and 110 are provided, and the number of the outlet portions 104 and 105 and the discharge flow paths 109 and 110 may be three or more.

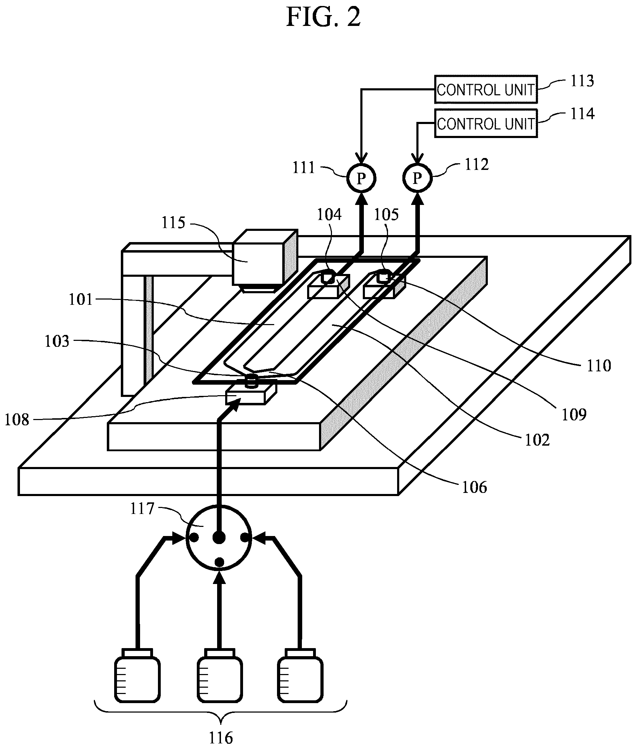

FIG. 2 shows a configuration diagram when the substrate 107 is mounted on the nucleic acid analyzer 100. The reagent selection mechanism 117 selects a reagent required for each reaction step. Next, the reagent aspiration mechanisms 111 and 112 aspirate the reagent. The amount of reagent flowing into the flow path 101 is controlled by the control unit 113, and the amount of reagent flowing into the flow path 102 is controlled by the control unit 114. By independently controlling a reagent aspirating amount by each reagent aspiration mechanism in such a manner, it is possible to introduce a desired reagent amount into each flow path.

As described above, in the substrate having a plurality of flow paths, the number of the introduction flow paths 108 can be minimized by providing the flow path branch point 106 on the substrate. Therefore, even when a branched flow path structure is provided, it is not necessary to replace a branched flow path on a device side with a reagent as in the related art, and thus it is possible to prevent excessive reagents from being consumed.

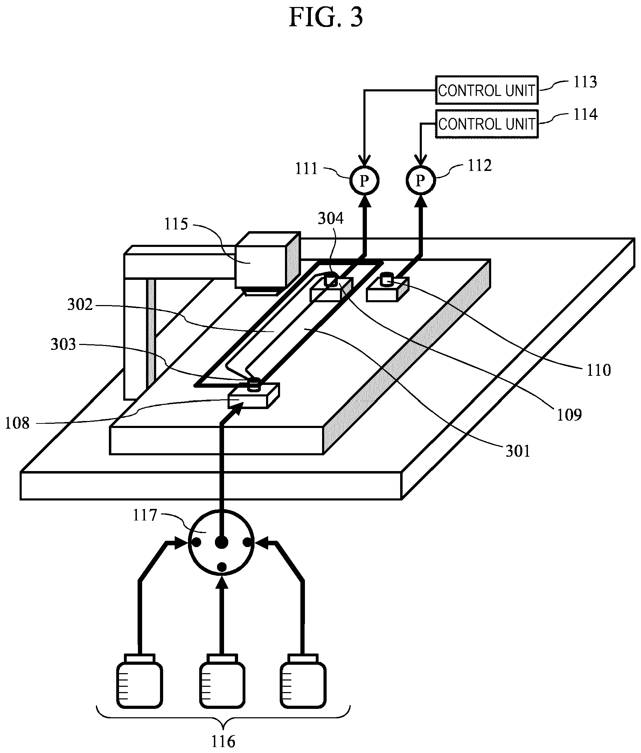

FIG. 3 shows a configuration diagram when a substrate 301 having a size different from that of the substrate 107 is mounted on the nucleic acid analyzer 100. The substrate 301 includes one flow path 302, one inlet portion 303, and one outlet portion 304. The inlet portion 303 is connected to the introduction flow path 108, and the outlet portion 304 is connected to the discharge flow path 109. The flow path 302 connects the inlet portion 303 and the outlet portion 304. The reagent aspiration mechanism 111 introduces the reagent into the flow path 302 of the substrate 301 via the reagent selection mechanism 117, the inlet portion 303, and the outlet portion 304.

As shown in FIG. 3, even when substrates 301 of different sizes are mounted, an unnecessary dead volume does not occur between the reagent containers 116 and the inlet portion 303. Therefore, contamination of the reagent and bubbles remaining in a branched flow path portion that is not connected (not used) to the substrate 301 as in the related art does not occur.

### <First Embodiment: Summary>

In the nucleic acid analyzer 100 according to the first embodiment, the substrate 107 includes the flow path branch point 106, and a reagent is branched from the flow path branch point 106 to each flow path on the substrate 107. In other words, the flow path branch point 106 is disposed on a substrate 107 side. Accordingly, it is sufficient to provide the minimum number of introduction flow paths 108 on the device side (if one inlet portion 103 is provided, one introduction flow path 108 is also provided). Therefore, it is not necessary to replace the branched flow path on the device side with the reagent as in the related art, and thus it is possible to prevent a reagent consumption amount.

In the nucleic acid analyzer 100 according to the first embodiment, even when the substrate 107 is replaced with the substrate 301, no branched flow path that is not connected to the substrate 301 is generated, and thus unnecessary dead volume does not occur between the reagent containers 116 and the inlet portion 303. Therefore, it is possible to prevent contamination of the reagent or the bubbles remaining in the branched flow path portion that is not connected (not used) to the substrate 301 as in the related art.

### <Second Embodiment>

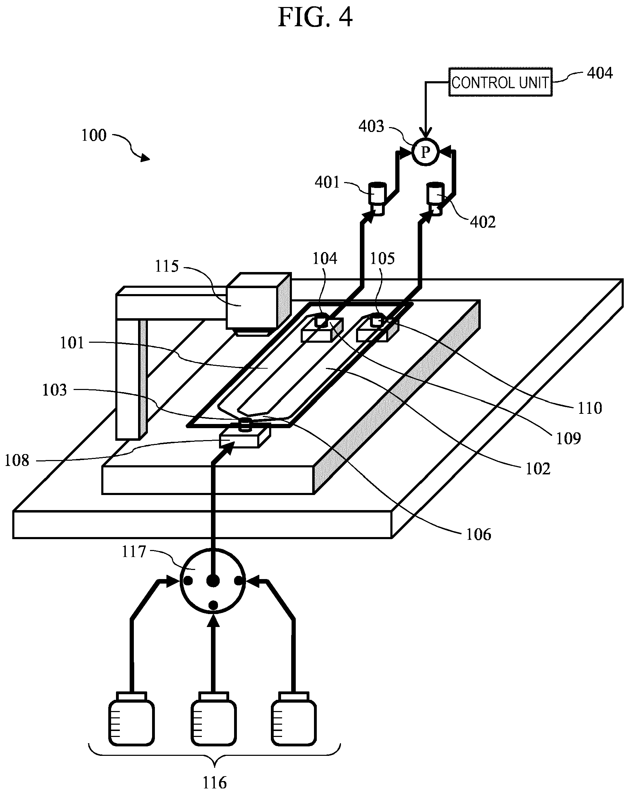

FIG. 4 shows a configuration diagram of the nucleic acid analyzer 100 according to a second embodiment of the invention. In FIG. 4, descriptions of parts having the same functions as those of the configuration shown in FIG. 1 will be omitted. In the second embodiment, the discharge flow path 109 is connected to a reagent aspiration mechanism 403 via a first solenoid valve 401, and the discharge flow path 110 is connected to a reagent aspiration mechanism 403 via a second solenoid valve 402. Other configurations are the same as those according to the first embodiment.

In the second embodiment, the control unit 404 (a) opens the first solenoid valve 401 when a desired amount of reagent is to be introduced into the flow path 101, and controls the reagent aspiration mechanism 403 via the control unit 404 of the reagent aspiration mechanism in a state where the second solenoid valve 402 is closed, and (b) opens the second solenoid valve 402 when a desired amount of reagent is to be introduced into the flow path 102, and controls the reagent aspiration mechanism 403 in a state where the first solenoid valve 401 is closed.

Specifically, when the substrate 107 is used, the first solenoid valve 401 is opened to introduce the reagent, and then the second solenoid valve 402 is opened to introduce the reagent; when the substrate 301 is used, only the first solenoid valve 401 is opened to introduce the reagent.

The nucleic acid analyzer 100 according to the second embodiment can reduce the number of reagent aspiration mechanisms and the number of control units as compared with the first embodiment. Accordingly, it is possible to simplify a structure particularly after the discharge flow paths.

In FIG. 4, two solenoid valves are provided in order to select a flow path for discharging the reagent, but the flow path can also be selected using one three-way solenoid valve. In addition, the discharge flow path may be selected by other appropriate mechanisms.

### <Third Embodiment>

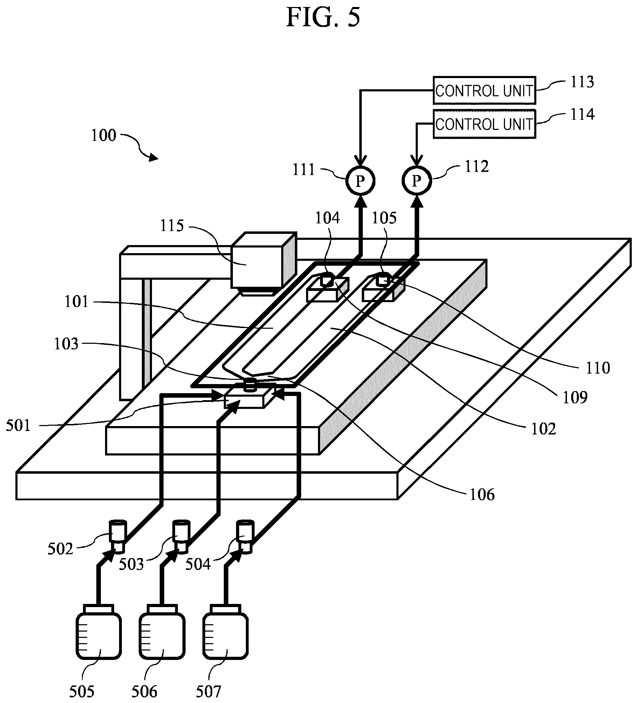

FIG. 5 shows a configuration diagram of the nucleic acid analyzer 100 according to a third embodiment of the invention. In FIG. 5, descriptions of parts having the same functions as those of the configuration shown in FIG. 1 will be omitted. In the third embodiment, provided on a reagent introduction side are a block 501, a first reagent selection solenoid valve 502, a second reagent selection solenoid valve 503, a third reagent selection solenoid valve 504, a first reagent container 505, a second reagent container 506, and a third reagent container 507. Other configurations are the same as those according to the first embodiment.

The block 501 includes a plurality of branched flow paths connected to the inlet portion 103. When the first reagent container 505 is connected to the substrate 107, the reagent is aspirated by the reagent aspiration mechanisms 111 and 112 in a state where the first reagent selection solenoid valve 502 is opened and the second reagent selection solenoid valve 503 and the third reagent selection solenoid valve 504 are closed. Similarly, when the second reagent container 506 and the third reagent container 507 are connected to the substrate 107, a desired reagent is selectively introduced into the substrate 107 by opening the corresponding second reagent selection solenoid valve 503 or the corresponding third reagent selection solenoid valve 504. The same applies to a case of using the substrate 301.

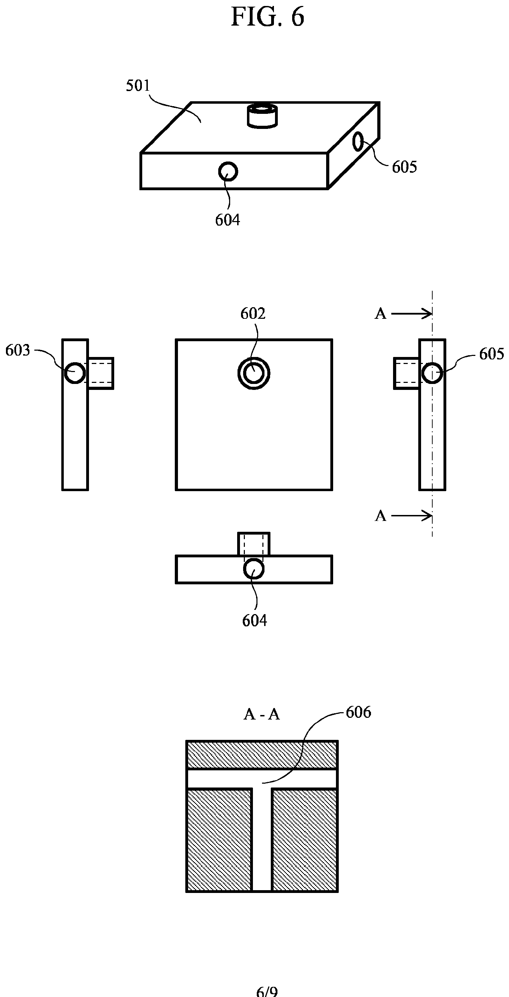

FIG. 6 is a configuration diagram of the block 501. An upper part of FIG. 6 is a perspective view, a middle part of FIG. 6 includes a top view, left and right side views, and a front view, and a lower part of FIG. 6 is a cross-sectional view taken along line AA. The block 501 includes an outflow port 602 through which reagents flow out to a substrate and in contact with a flow path of the substrate, a first reagent inflow port 603, a second reagent inflow port 604, and a third reagent inflow port 605.

In the block 501, the branched flow paths from reagent inflow ports merge at a merging point 606 and reach the outflow port 602 through which the reagents flow out to the substrate. Although three reagent inflow ports are provided in FIG. 6, the number of reagent inflow ports may be increased or decreased according to the number of reagents required for reaction, and the number of merging points 606 may also be increased or decreased accordingly.

### <Fourth Embodiment>

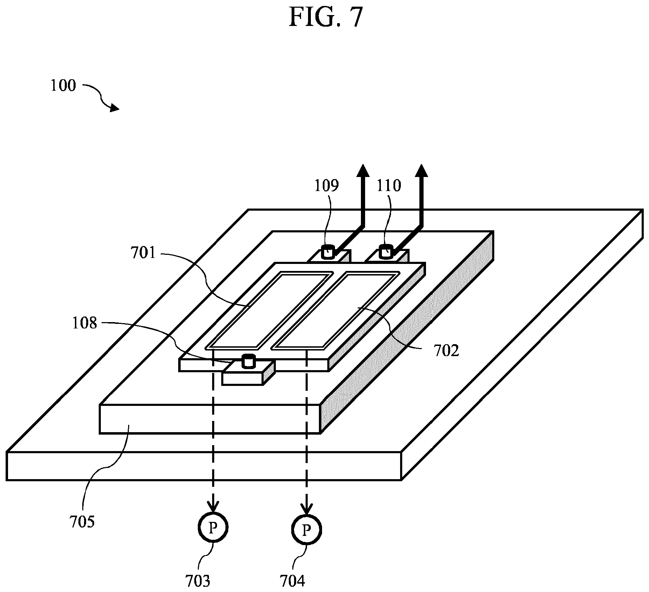

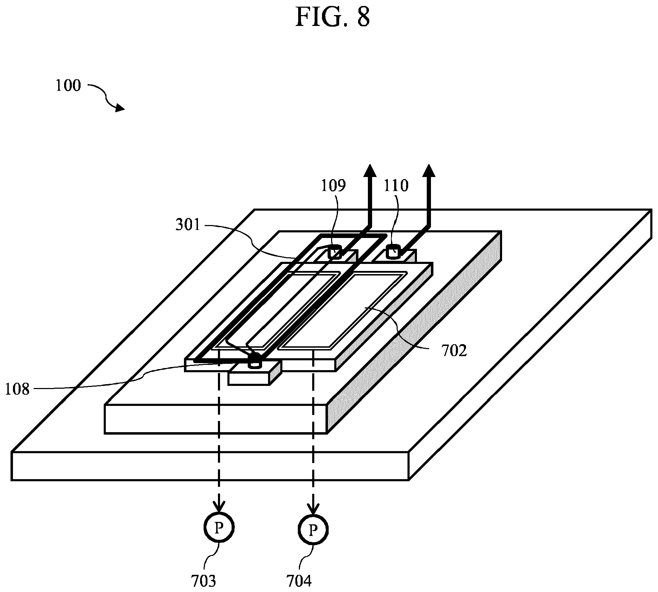

FIG. 7 is a configuration diagram of the nucleic acid analyzer 100 according to a fourth embodiment of the invention. In the fourth embodiment, the nucleic acid analyzer 100 includes grooves 701 and 702 on a stage 705 on which a substrate is placed. The groove 701 is connected to a pump 703, and the pump 703 evacuates the groove 701. The groove 702 is connected to a pump 704, and the pump 704 evacuates the groove 702. The pumps 703 and 704 can be controlled by, for example, the control units 113 and 114, respectively. Other configurations are the same as those according to the first to third embodiments, and thus descriptions thereof are omitted in FIG. 7. The same applies to FIG. 8.

FIG. 8 is a diagram showing a state in which the substrate 301 is used. When placed on the stage 705, the substrate 301 has a size and a shape to cover the groove 701 while not overlapping the groove 702. When the substrate 301 is used, the substrate 301 is placed on the groove 701, and the pump 703 aspirates the substrate 301 via the groove 701. Accordingly, the substrate 301 can be fixed on the stage.

When placed on the stage 705, the substrate 107 has a size and a shape to cover both the grooves 701 and 702. Similarly, when the substrate 107 is used, the substrate 107 is placed on the grooves 701 and 702, and the pumps 703 and 704 aspirate the substrate 107 via the grooves 701 and 702, respectively. Accordingly, the substrate 107 can be fixed on the stage.

As in the fourth embodiment, a mechanism that fixes the substrate is divided into a plurality of (two grooves 701 and 702 in FIG. 7) mechanisms, and which fixing mechanism is used is switched according to the size of the substrate. Accordingly, it is possible to flexibly use substrates of various sizes and to reliably fix any substrate.

Although two pumps 703 and 704 are shown in FIG. 7, one pump and a solenoid valve may be used to switch which groove is to be aspirated. Therefore, the number of pumps is set for the sake of convenience as long as which groove is to be aspirated can be switched according to the size of the substrate.

In FIG. 7, the grooves 701 and 702 are disposed on the stage 705, but positions of the grooves are not limited thereto, and may be any positions as long as the substrate covers these grooves when the substrate 107 or the substrate 301 is mounted on the nucleic acid analyzer 100.

### <Fifth Embodiment>

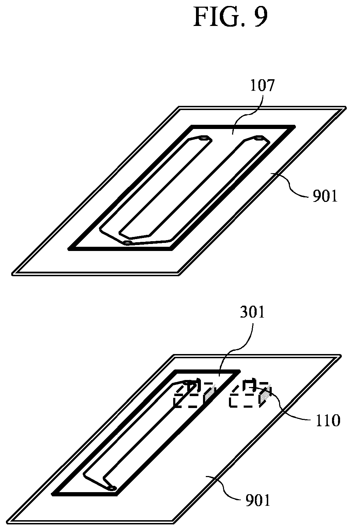

FIG. 9 is a configuration diagram of the substrates 107 and 301 used in the nucleic acid analyzer 100 according to a fifth embodiment of the invention. Each substrate can be accommodated in a casing 901. The casing 901 may be shared between the substrates 107 and 301, and may be provided with separate substrates having different sizes and made of different materials.

The casing 901 has a planar size slightly larger than the substrate 301. Accordingly, when the substrate 301 is accommodated in the casing 901 and placed on the stage 705, the discharge flow path 110 on a not-used side can be closed. If the discharge flow path 110 is opened for a long time (for example, from about several hours to about several days) without being used, dust or the like may clog the inside of the discharge flow path 110. By attaching the casing 901, such clogging can be prevented even when the substrate 107 is replaced with the substrate 301.

### <Modifications of Invention>

The invention is not limited to the embodiments described above, and includes various modifications. For example, the above-described embodiments are described in detail for easy understanding of the invention, and the invention is not necessarily limited to those including all the configurations described above. Further, a part of a configuration according to one embodiment can be replaced with a configuration according to another embodiment, and the configuration according to another embodiment can be added to the configuration according to one embodiment. A part of a configuration according to each embodiment can be added, deleted, or replaced with another configuration, as long as the resulting configuration is according to the claims.

In the above embodiments, an imaging range of the imaging mechanism 115 when the substrate 107 is used is larger than that when the substrate 301 is used. Therefore, when the substrate is moved within the imaging range of the imaging mechanism 115, a moving range is larger when the substrate 107 is used. For example, when the substrate is placed on the stage 705 and moved with the stage 705, a moving range of the stage 705 is larger when the substrate 107 is used. Alternatively, if the imaging mechanism 115 can scan an imaging range or an imaging location, the imaging range is larger when the substrate 107 is used.

In the embodiments described above, the control units 113, 114, and 404 can be implemented by hardware such as a circuit device in which functions of the control units are implemented, and can be implemented by an arithmetic device executing software in which the functions of the control units are implemented.

### Reference Signs List

100 nucleic acid analyzer
101 flow path
102 flow path
103 inlet portion
104 outlet portion
105 outlet portion
106 flow path branch point
107 substrate
108 introduction flow path
109 discharge flow path
110 discharge flow path
111 reagent aspiration mechanism
112 reagent aspiration mechanism
113 control unit
114 control unit
115 imaging mechanism
116 reagent container
117 reagent selection mechanism
301 substrate
302 flow path
303 inlet portion
304 outlet portion
401 first solenoid valve
402 second solenoid valve
403 reagent aspiration mechanism
404 control unit
501 block
502 first reagent selection solenoid valve
503 second reagent selection solenoid valve
504 third reagent selection solenoid valve
505 first reagent container
506 second reagent container
507 third reagent container
602 outflow port
603 first reagent inflow port
604 second reagent inflow port
605 third reagent inflow port
606 merging point
701 groove
702 groove
703 pump
704 pump
705 stage
901 casing

## Claims

1. A nucleic acid analyzer comprising:
a first substrate (107) including a reaction field for analyzing a nucleic acid;
an introduction path (108) through which a reagent to be introduced into the first substrate (107) is conveyed;
a first discharge path (109) through which the reagent discharged from the first substrate (107) is conveyed;
a second discharge path (110) through which the reagent discharged from the first substrate (107) is conveyed;
a reagent selection mechanism (117; 502~504) configured to select the reagent to be introduced into the first substrate (107); and
an aspiration mechanism (111, 112; 401~403) configured to aspirate the reagent from upstream sides of the first discharge path (109) and the second discharge path (110) to introduce the reagent into the first substrate (107) via the introduction path (108), wherein the nucleic acid analyzer, in addition to the first substrate (107), further comprises a second substrate (301), and is capable of exchanging the first substrate (107) and the second substrate (301), wherein
the first substrate (107) includes:
an inlet portion (103) connectable to the introduction path (108);
a first outlet portion (104) connectable to the first discharge path (109);
a second outlet portion (105) connectable to the second discharge path (110);
a first flow path (101) configured to guide the reagent from the inlet portion (103) to the first outlet portion (104);
a second flow path (102) configured to guide the reagent from the inlet portion (103) to the second outlet portion (105); and
a branch portion (106) configured to branch the reagent from the inlet portion (103) to the first flow path (101) and the second flow path (102), and
the first flow path (101) and the second flow path (102) are connected to each other only at the branch portion (106),
**characterised in that**
the second substrate (301) includes:
a single third inlet portion (303) connectable to the introduction path (108);
a single third outlet portion (304) connectable to the first discharge path (109); and
a single third flow path (302) configured to guide the reagent from the single third inlet portion (303) to the single third outlet portion (304),
when the first substrate (107) is used, the aspiration mechanism (111, 112; 401~403) is configured to aspirate the reagent via the first flow path (101), the first outlet portion (104), and the first discharge path (109), and aspirates the reagent via the second flow path (102), the second outlet portion (105), and the second discharge path (110), and
when the second substrate (301) is used, the aspiration mechanism (111, 112; 401~403) is configured to aspirate the reagent via the single third flow path (302) and the single third outlet portion (304) and via one of the first discharge path (109) and the second discharge path (110).

2. The nucleic acid analyzer according to claim 1, further comprising:
a connection flow path configured to connect a reagent container (116) containing the reagent to the introduction path (108), wherein
the connection flow path does not branch along the way from the reagent container (116) to the introduction path (108).

3. The nucleic acid analyzer according to claim 1, further comprising:
a stage (705) on which a substate can be placed;
a first groove (701) located on the stage (705) below the first substrate (107) when the first substrate is attached to the nucleic acid analyzer; and
a first pump (703) configured to aspirate the first substrate (107) via the first groove (701), wherein
the first pump (703) fixes the first substrate (107) to the nucleic acid analyzer by aspirating the first substrate (107) via the first groove (701).

4. The nucleic acid analyzer according to claim 1, further comprising:
a stage (705) on which a substate can be placed;
a first groove (701) located on the stage (705) below the first substrate (107) when the first substrate is attached to the nucleic acid analyzer;
a second groove (702) located on the stage (705) below the first substrate (107) when the first substrate is attached to the nucleic acid analyzer;
a first pump (703) configured to aspirate the first substrate (107) via the first groove (701); and
a second pump (704) configured to aspirate the first substrate (107) via the second groove (702), wherein
the first substrate (107) has a shape and a planar size such that both the first groove (701) and the second groove (702) are covered with the first substrate when the first substrate is attached to the nucleic acid analyzer,
the second substrate (301) has a shape and a planar size such that the first groove (701) is covered with the second substrate and the second groove (704) is not covered with the second substrate when the second substrate is attached to the nucleic acid analyzer,
the first pump (703) and the second pump (704) fix the first substrate (107) to the nucleic acid analyzer by aspirating the first substrate via the first groove (701) and the second groove (702), respectively, and
the first pump (703) fixes the second substrate (301) to the nucleic acid analyzer by aspirating the second substrate via the first groove (701).

5. The nucleic acid analyzer according to claim 1, further comprising:
a casing (901) accommodating the second substrate (301), wherein
the casing (901) has a shape and a size to close the second discharge path (110) when the second substrate (301) is attached to the nucleic acid analyzer.

6. The nucleic acid analyzer according to claim 1, further comprising:
an imaging mechanism (115) configured to image a specimen flowing through the first substrate (107) or the second substrate (301), wherein
a range in which the imaging mechanism (115) captures an image when the first substrate (107) is attached to the nucleic acid analyzer is larger than a range in which the imaging mechanism (115) captures an image when the second substrate (301) is attached to the nucleic acid analyzer.

7. The nucleic acid analyzer according to claim 1, wherein
the aspiration mechanism (111, 112) includes:
a first aspiration unit (111) connected to the first discharge path (109); and
a second aspiration unit (112) connected to the second discharge path (110),
the first aspiration unit (111) aspirates the reagent via the first discharge path (109), and the second aspiration unit (112) aspirates the reagent via the second discharge path (110) to introduce the reagent into the first substrate (107), and
the first aspiration unit (111) aspirates the reagent via the first discharge path (109) to introduce the reagent into the second substrate (301).

8. The nucleic acid analyzer according to claim 1, wherein
the aspiration mechanism (401~403) includes:
an aspiration unit (403) connected to the first discharge path (109) and the second discharge path (110);
a first valve (401) configured to block or open the first discharge path (109); and
a second valve (402) configured to block or open the second discharge path (110),
the first valve (401) opens the first discharge path (109), the second valve (402) blocks the second discharge path (110), and the aspiration unit (403) aspirates the reagent to introduce the reagent into the first flow path (101),
the first valve (401) blocks the first discharge path (109), the second valve (402) opens the second discharge path (110), and the aspiration unit (403) aspirates the reagent to introduce the reagent into the second flow path (102), and
one of the first valve (401) and the second valve (402) is opened while the other valve is blocked, and the aspiration unit (403) aspirates the reagent to introduce the reagent into the third flow path (302).

9. The nucleic acid analyzer according to claim 1, wherein
the reagent selection mechanism (117; 502~504) includes:
a first branch path connected to a first reagent container (116; 505~507) containing a first reagent;
a second branch path connected to a second reagent container (116; 505~507) containing a second reagent;
a merging point at which the first branch path and the second branch path merge; and
a flow path configured to connect the merging point and the inlet portion (103).

10. The nucleic acid analyzer according to claim 9, wherein
the reagent selection mechanism (502~504) includes:
a third valve (502~504) configured to block or open a flow path between the first branch path and the first reagent container (505~507); and
a fourth valve (502~504) configured to block or open a flow path between the second branch path and the second reagent container (116; 505~507),
the reagent selection mechanism selects the first reagent by opening the third valve and blocking the fourth valve, and
the reagent selection mechanism (117) selects the second reagent by blocking the third valve (502~504) and opening the fourth valve (502~504).

11. The nucleic acid analyzer according to claim 1, wherein
the number of paths (109, 110) configured to convey the reagent discharged from the first substrate (107) is larger than the number of introduction paths (108).

## Patentansprüche

1. Nukleinsäure-Analysegerät umfassend:
ein erstes Substrat (107), das ein Reaktionsfeld zur Analyse einer Nukleinsäure enthält;
einen Einführungspfad (108), durch den ein in das erste Substrat (107) einzuführendes Reagens befördert wird;
einen ersten Abgabepfad (109), durch den das von dem ersten Substrat (107) abgegebene Reagens befördert wird;
einen zweiten Abgabepfad (110), durch den das von dem ersten Substrat (107) abgegebene Reagens befördert wird;
einen Reagenzienauswahlmechanismus (117; 502~504), der dazu konfiguriert ist, das in das erste Substrat (107) einzuführende Reagens auszuwählen; und
einen Ansaugmechanismus (111, 112; 401~403), der dazu konfiguriert ist, das Reagens von stromaufwärts gelegenen Seiten des ersten Abgabepfads (109) und des zweiten Abgabepfads (110) anzusaugen, um das Reagens über den Einführungspfad (108) in das erste Substrat (107) einzuführen, wobei das Nukleinsäure-Analysegerät zusätzlich zu dem ersten Substrat (107) ferner ein zweites Substrat (301) umfasst und dazu in der Lage ist, das erste Substrat (107) und das zweite Substrat (301) auszutauschen, wobei
das erste Substrat (107) umfasst:
einen Einlassabschnitt (103), der mit dem Einführungspfad (108) verbunden werden kann;
einen ersten Auslassabschnitt (104), der mit dem ersten Abgabepfad (109) verbunden werden kann;
einen zweiten Auslassabschnitt (105), der mit dem zweiten Abgabepfad (110) verbunden werden kann;
einen ersten Strömungspfad (101), der dazu konfiguriert ist, das Reagens von dem Einlassabschnitt (103) zu dem ersten Auslassabschnitt (104) zu leiten;
einen zweiten Strömungspfad (102), der dazu konfiguriert ist, das Reagens von dem Einlassabschnitt (103) zu dem zweiten Auslassabschnitt (105) zu leiten; und
einen Verzweigungsabschnitt (106), der dazu konfiguriert ist, das Reagens von dem Einlassabschnitt (103) zu dem ersten Strömungspfad (101) und zu dem zweiten Strömungspfad (102) abzuzweigen, und
der erste Strömungspfad (101) und der zweite Strömungspfad (102) nur an dem Verzweigungsabschnitt (106) miteinander verbunden sind,
**dadurch gekennzeichnet, dass**
das zweite Substrat (301) umfasst:
einen einzelnen dritten Einlassabschnitt (303), der mit dem Einführungspfad (108) verbunden werden kann;
einen einzelnen dritten Auslassabschnitt (304), der mit dem ersten Abgabepfad (109) verbunden werden kann; und
einen einzelnen dritten Strömungspfad (302), der dazu konfiguriert ist, das Reagens von dem einzelnen dritten Einlassabschnitt (303) zu dem einzelnen dritten Auslassabschnitt (304) zu leiten,
wenn das erste Substrat (107) verwendet wird, der Ansaugmechanismus (111, 112; 401~403) dazu konfiguriert ist, das Reagens über den ersten Strömungspfad (101), den ersten Auslassabschnitt (104) und den ersten Abgabepfad (109) anzusaugen, und das Reagens über den zweiten Strömungspfad (102), den zweiten Auslassabschnitt (105) und den zweiten Abgabepfad (110) anzusaugen, und
wenn das zweite Substrat (301) verwendet wird, der Ansaugmechanismus (111, 112; 401~403) dazu konfiguriert ist, das Reagens über den einzelnen dritten Strömungspfad (302) und den einzelnen dritten Auslassabschnitt (304) und über einen von dem ersten Abgabepfad (109) und dem zweiten Abgabepfad (110) anzusaugen.

2. Nukleinsäure-Analysegerät nach Anspruch 1, ferner umfassend:
einen Verbindungsströmungspfad, der dazu konfiguriert ist, einen Reagenzienbehälter (116), der das Reagens enthält, mit dem Einführungspfad (108) zu verbinden,
wobei sich der Verbindungsströmungspfad auf dem Weg von dem Reagenzienbehälter (116) zu dem Einführungspfad (108) nicht verzweigt.

3. Nukleinsäure-Analysegerät nach Anspruch 1, ferner umfassend:
einen Tisch (705), auf dem ein Substrat platziert werden kann;
eine erste Nut (701), die sich auf dem Tisch (705) unterhalb des ersten Substrats (107) befindet, wenn das erste Substrat an dem Nukleinsäure-Analysegerät angebracht ist; und
eine erste Pumpe (703), die dazu konfiguriert ist, das erste Substrat (107) über die erste Nut (701) anzusaugen,
wobei die erste Pumpe (703) das erste Substrat (107) an dem Nukleinsäure-Analysegerät fixiert, indem sie das erste Substrat (107) über die erste Nut (701) ansaugt.

4. Nukleinsäure-Analysegerät nach Anspruch 1, ferner umfassend:
einen Tisch (705), auf dem ein Substrat platziert werden kann;
eine erste Nut (701), die sich auf dem Tisch (705) unterhalb des ersten Substrats (107) befindet, wenn das erste Substrat an dem Nukleinsäure-Analysegerät angebracht ist;
eine zweite Nut (702), die sich auf dem Tisch (705) unterhalb des ersten Substrats (107) befindet, wenn das erste Substrat an dem Nukleinsäure-Analysegerät angebracht ist;
eine erste Pumpe (703), die dazu konfiguriert ist, das erste Substrat (107) über die erste Nut (701) anzusaugen, und
eine zweite Pumpe (704), die dazu konfiguriert ist, das erste Substrat (107) über die zweite Nut (702) anzusaugen, wobei
das erste Substrat (107) eine solche Form und ebene Größe aufweist, dass sowohl die erste Nut (701) als auch die zweite Nut (702) mit dem ersten Substrat bedeckt sind, wenn das erste Substrat an dem Nukleinsäure-Analysegerät angebracht ist,
das zweite Substrat (301) eine solche Form und ebene Größe aufweist, dass die erste Nut (701) mit dem zweiten Substrat bedeckt ist und die zweite Nut (702) nicht mit dem zweiten Substrat bedeckt ist, wenn das zweite Substrat an dem Nukleinsäure-Analysegerät angebracht ist,
die erste Pumpe (703) und die zweite Pumpe (704) das erste Substrat (107) durch Ansaugen des ersten Substrats über die erste Nut (701) bzw. die zweite Nut (702) an dem Nukleinsäure-Analysegerät befestigen, und
die erste Pumpe (703) das zweite Substrat (301) an dem Nukleinsäure-Analysegerät durch Ansaugen des zweiten Substrats über die erste Nut (701) befestigt.

5. Nukleinsäure-Analysegerät nach Anspruch 1, ferner umfassend:
ein Gehäuse (901), das das zweite Substrat (301) aufnimmt,
wobei das Gehäuse (901) eine Form und Größe aufweist, um den zweiten Abgabepfad (110) zu schließen, wenn das zweite Substrat (301) an dem Nukleinsäure-Analysegerät angebracht ist.

6. Nukleinsäure-Analysegerät nach Anspruch 1, ferner umfassend:
einen Abbildungsmechanismus (115), der dazu konfiguriert ist, eine durch das erste Substrat (107) oder das zweite Substrat (301) strömende Probe abzubilden,
wobei ein Bereich, in dem der Abbildungsmechanismus (115) ein Bild einfängt, wenn das erste Substrat (107) an dem Nukleinsäure-Analysegerät angebracht ist, größer ist als ein Bereich, in dem der Abbildungsmechanismus (115) ein Bild einfängt, wenn das zweite Substrat (301) an dem Nukleinsäure-Analysegerät angebracht ist.

7. Nukleinsäure-Analysegerät nach Anspruch 1, wobei
der Ansaugmechanismus (111, 112) umfasst:
eine erste Ansaugeinheit (111), die mit dem ersten Abgabepfad (109) verbunden ist; und
eine zweite Ansaugeinheit (112), die mit dem zweiten Abgabepfad (110) verbunden ist,
die erste Ansaugeinheit (111) das Reagens über den ersten Abgabepfad (109) ansaugt, und die zweite Ansaugeinheit (112) das Reagens über den zweiten Abgabepfad (110) ansaugt, um das Reagens in das erste Substrat (107) einzuführen, und
die erste Ansaugeinheit (111) das Reagens über den ersten Abgabepfad (109) ansaugt, um das Reagens in das zweite Substrat (301) einzuführen.

8. Nukleinsäure-Analysegerät nach Anspruch 1, wobei
der Ansaugmechanismus (401~ 403) umfasst:
eine Ansaugeinheit (403), die mit dem ersten Abgabepfad (109) und dem zweiten Abgabepfad (110) verbunden ist;
ein erstes Ventil (401), das dazu konfiguriert ist, den ersten Abgabepfad (109) zu sperren oder zu öffnen; und
ein zweites Ventil (402), das dazu konfiguriert ist, den zweiten Abgabepfad (110) zu sperren oder zu öffnen,
das erste Ventil (401) den ersten Abgabepfad (109) öffnet, das zweite Ventil (402) den zweiten Abgabepfad (110) sperrt und die Ansaugeinheit (403) das Reagens ansaugt, um es in den ersten Strömungspfad (101) einzuführen,
das erste Ventil (401) den ersten Abgabepfad (109) sperrt, das zweite Ventil (402) den zweiten Abgabepfad (110) öffnet und die Ansaugeinheit (403) das Reagens ansaugt, um das Reagens in den zweiten Strömungspfad (102) einzuführen, und
eines von dem ersten Ventil (401) und dem zweiten Ventil (402) geöffnet wird, während das andere Ventil gesperrt ist, und die Ansaugeinheit (403) das Reagens ansaugt, um das Reagens in den dritten Strömungspfad (302) einzuführen.

9. Nukleinsäure-Analysegerät nach Anspruch 1, wobei
der Reagenzienauswahlmechanismus (117; 502~504) umfasst:
einen ersten Verzweigungspfad, der mit einem ersten Reagenzienbehälter (116; 505~507) verbunden ist, der ein erstes Reagens enthält;
einen zweiten Verzweigungspfad, der mit einem zweiten Reagenzienbehälter (116; 505~507) verbunden ist, der ein zweites Reagens enthält;
einen Zusammenführungspunkt, an dem der erste Verzweigungspfad und der zweite Verzweigungspfad zusammenlaufen; und
einen Strömungspfad, der dazu konfiguriert ist, den Zusammenführungspunkt und den Einlassabschnitt (103) zu verbinden.

10. Nukleinsäure-Analysegerät nach Anspruch 9, wobei
der Reagenzienauswahlmechanismus (502~504) umfasst:
ein drittes Ventil (502-504), das dazu konfiguriert ist, einen Strömungspfad zwischen dem ersten Verzweigungspfad und dem ersten Reagenzienbehälter (505~507) zu sperren oder zu öffnen; und
ein viertes Ventil (502-504), das dazu konfiguriert ist, einen Strömungspfad zwischen dem zweiten Verzweigungspfad und dem zweiten Reagenzienbehälter (116; 505~507) zu sperren oder zu öffnen,
der Reagenzienauswahlmechanismus das erste Reagens auswählt, indem er das dritte Ventil öffnet und das vierte Ventil sperrt, und
der Reagenzienauswahlmechanismus (117) das zweite Reagens auswählt, indem er das dritte Ventil (502~504) sperrt und das vierte Ventil (502~504) öffnet.

11. Nukleinsäure-Analysegerät nach Anspruch 1, wobei die Anzahl der Pfade (109, 110), die dazu konfiguriert sind, das von dem ersten Substrat (107) abgegebene Reagens zu befördern, größer ist als die Anzahl der Einführungspfade (108).

## Revendications

1. Analyseur d'acide nucléique comprenant :
un premier substrat (107) incluant un champ de réaction pour analyser un acide nucléique ;
un chemin (108) d'introduction à travers lequel un réactif à introduire dans le premier substrat (107) est transporté ;
un premier chemin (109) de décharge à travers lequel le réactif déchargé du premier substrat (107) est transporté ;
un deuxième chemin (110) de décharge à travers lequel le réactif déchargé du premier substrat (107) est transporté ;
un mécanisme (117 ; 502~504) de sélection de réactif configuré pour sélectionner le réactif à introduire dans le premier substrat (107) ; et
un mécanisme (111, 112 ; 401~403) d'aspiration configuré pour aspirer le réactif depuis des côtés amont du premier chemin (109) de décharge et du deuxième chemin (110) de décharge pour introduire le réactif dans le premier substrat (107) par le chemin (108) d'introduction,
dans lequel l'analyseur d'acide nucléique, en plus du premier substrat (107), comprend en outre un deuxième substrat (301), et est apte à échanger le premier substrat (107) et le deuxième substrat (301), dans lequel
le premier substrat (107) inclut :
une partie (103) d'entrée connectable au chemin (108) d'introduction ;
une première partie (104) de sortie connectable au premier chemin (109) de décharge ;
une deuxième partie (105) de sortie connectable au deuxième chemin (110) de décharge ;
un premier chemin (101) d'écoulement configuré pour guider le réactif de la partie (103) d'entrée à la première partie (104) de sortie ;
un deuxième chemin (102) d'écoulement configuré pour guider le réactif de la partie (103) d'entrée à la deuxième partie (105) de sortie ; et
une partie (106) de dérivation configurée pour dériver le réactif de la partie (103) d'entrée au premier chemin (101) d'écoulement et au deuxième chemin (102) d'écoulement, et
le premier chemin (101) d'écoulement et le deuxième chemin (102) d'écoulement sont connectés l'un à l'autre uniquement au niveau de la partie (106) de dérivation,
**caractérisé en ce que**
le deuxième substrat (301) inclut :
une troisième partie (303) d'entrée unique connectable au chemin (108) d'introduction ;
une troisième partie (304) de sortie unique connectable au premier chemin (109) de décharge ; et
un troisième chemin (302) d'écoulement unique configuré pour guider le réactif de la troisième partie (303) d'entrée unique à la troisième partie (304) de sortie unique,
lorsque le premier substrat (107) est utilisé, le mécanisme (111, 112 ; 401~403) d'aspiration est configuré pour aspirer le réactif par l'intermédiaire du premier chemin (101) d'écoulement, de la première partie (104) de sortie, et du premier chemin (109) de décharge, et aspire le réactif par l'intermédiaire du deuxième chemin (102) d'écoulement, de la deuxième partie (105) de sortie, et du deuxième chemin (110) de décharge, et
lorsque le deuxième substrat (301) est utilisé, le mécanisme (111, 112 ; 401~403) d'aspiration est configuré pour aspirer le réactif par l'intermédiaire du troisième chemin (302) d'écoulement unique et de la troisième partie (304) de sortie unique et par l'intermédiaire d'un du premier chemin (109) de décharge et du deuxième chemin (110) de décharge.

2. Analyseur d'acide nucléique selon la revendication 1, comprenant en outre :
un chemin d'écoulement de connexion configuré pour connecter un récipient (116) pour réactif contenant le réactif au chemin (108) d'introduction, dans lequel
le chemin d'écoulement de connexion ne dérive pas sur le chemin du récipient (116) pour réactif au chemin (108) d'introduction.

3. Analyseur d'acide nucléique selon la revendication 1, comprenant en outre :
une platine (705) sur laquelle un substrat peut être placé ;
une première rainure (701) située sur la platine (705) en-dessous du premier substrat (107) lorsque le premier substrat est fixé à l'analyseur d'acide nucléique ; et
une première pompe (703) configurée pour aspirer le premier substrat (107) par l'intermédiaire de la première rainure (701), dans lequel
la première pompe (703) fixe le premier substrat (107) à l'analyseur d'acide nucléique en aspirant le premier substrat (107) par l'intermédiaire de la première rainure (701).

4. Analyseur d'acide nucléique selon la revendication 1, comprenant en outre :
une platine (705) sur laquelle un substrat peut être placé ;
une première rainure (701) située sur la platine (705) en-dessous du premier substrat (107) lorsque le premier substrat est fixé à l'analyseur d'acide nucléique ;
une deuxième rainure (702) située sur la platine (705) en-dessous du premier substrat (107) lorsque le premier substrat est fixé à l'analyseur d'acide nucléique ;
une première pompe (703) configurée pour aspirer le premier substrat (107) par l'intermédiaire de la première rainure (701) ; et
une deuxième pompe (704) configurée pour aspirer le premier substrat (107) par l'intermédiaire de la deuxième rainure (702), dans lequel
le premier substrat (107) a une forme et une taille planaire telles que la première rainure (701) et la deuxième rainure (702) sont toutes les deux recouvertes avec le premier substrat lorsque le premier substrat est fixé à l'analyseur d'acide nucléique,
le deuxième substrat (301) a une forme et une taille planaire telles que la première rainure (701) est recouverte avec le deuxième substrat et la deuxième rainure (702) n'est pas recouverte avec le deuxième substrat lorsque le deuxième substrat est fixé à l'analyseur d'acide nucléique,
la première pompe (703) et la deuxième pompe (704) fixent le premier substrat (107) à l'analyseur d'acide nucléique en aspirant le premier substrat par l'intermédiaire de la première rainure (701) et de la deuxième rainure (702), respectivement, et
la première pompe (703) fixe le deuxième substrat (301) à l'analyseur d'acide nucléique en aspirant le deuxième substrat par l'intermédiaire de la première rainure (701).

5. Analyseur d'acide nucléique selon la revendication 1, comprenant en outre :
un boîtier (901) recevant le deuxième substrat (301), dans lequel
le boîtier (901) a une forme et une taille pour fermer le deuxième chemin (110) de décharge lorsque le deuxième substrat (301) est fixé à l'analyseur d'acide nucléique.

6. Analyseur d'acide nucléique selon la revendication 1, comprenant en outre :
un mécanisme (115) d'imagerie configuré pour imager un échantillon s'écoulant à travers le premier substrat (107) ou le deuxième substrat (301), dans lequel
une plage dans laquelle le mécanisme (115) d'imagerie capture une image lorsque le premier substrat (107) est fixé à l'analyseur d'acide nucléique est plus grande qu'une plage dans laquelle le mécanisme (115) d'imagerie capture une image lorsque le deuxième substrat (301) est fixé à l'analyseur d'acide nucléique.

7. Analyseur d'acide nucléique selon la revendication 1, dans lequel
le mécanisme (111, 112) d'aspiration inclut :
une première unité (111) d'aspiration connectée au premier chemin (109) de décharge ; et
une deuxième unité (112) d'aspiration connectée au deuxième chemin (110) de décharge,
la première unité (111) d'aspiration aspire le réactif par l'intermédiaire du premier chemin (109) de décharge, et la deuxième unité (112) d'aspiration aspire le réactif par l'intermédiaire du deuxième chemin (110) de décharge pour introduire le réactif dans le premier substrat (107), et
la première unité (111) d'aspiration aspire le réactif par l'intermédiaire du premier chemin (109) de décharge pour introduire le réactif dans le deuxième substrat (301).

8. Analyseur d'acide nucléique selon la revendication 1, dans lequel
le mécanisme (401~403) d'aspiration inclut :
une unité (403) d'aspiration connectée au premier chemin (109) de décharge et au deuxième chemin (110) de décharge ;
une première vanne (401) configurée pour bloquer ou ouvrir le premier chemin (109) de décharge ; et
une deuxième vanne (402) configurée pour bloquer ou ouvrir le deuxième chemin (110) de décharge,
la première vanne (401) ouvre le premier chemin (109) de décharge, la deuxième vanne (402) bloque le deuxième chemin (110) de décharge et l'unité (403) d'aspiration aspire le réactif pour introduire le réactif dans le premier chemin (101) d'écoulement,
la première vanne (401) bloque le premier chemin (109) de décharge, la deuxième vanne (402) ouvre le deuxième chemin (110) de décharge, et l'unité (403) d'aspiration aspire le réactif pour introduire le réactif dans le deuxième chemin (102) d'écoulement, et
une de la première vanne (401) et de la deuxième vanne (402) est ouverte tandis que l'autre vanne est bloquée, et l'unité (403) d'aspiration aspire le réactif pour introduire le réactif dans le troisième chemin (302) d'écoulement.

9. Analyseur d'acide nucléique selon la revendication 1, dans lequel
le mécanisme (117 ; 502~504) de sélection de réactif inclut :
un premier chemin de dérivation connecté à un premier récipient (116 ; 505~507) pour réactif contenant un premier réactif ;
un deuxième chemin de dérivation connecté à un deuxième récipient (116 ; 505~507) pour réactif contenant un deuxième réactif ;
un point de réunion auquel le premier chemin de dérivation et le deuxième chemin de dérivation se réunissent ; et
un chemin d'écoulement configuré pour connecter le point de réunion et la partie (103) d'entrée.

10. Analyseur d'acide nucléique selon la revendication 9, dans lequel
le mécanisme (502~504) de sélection de réactif inclut :
une troisième vanne (502~504) configurée pour bloquer ou ouvrir un chemin d'écoulement entre le premier chemin de dérivation et le premier récipient (505~507) pour réactif ; et
une quatrième vanne (502~504) configurée pour bloquer ou ouvrir un chemin d'écoulement entre le deuxième chemin de dérivation et le deuxième récipient (116 ; 505~507) pour réactif,
le mécanisme de sélection de réactif sélectionne le premier réactif en ouvrant la troisième vanne et en bloquant la quatrième vanne, et
le mécanisme (117) de sélection de réactif sélectionne le deuxième réactif en bloquant la troisième vanne (502~504) et en ouvrant la quatrième vanne (502~504).

11. Analyseur d'acide nucléique selon la revendication 1, dans lequel
le nombre de chemins (109, 110) configurés pour transporter le réactif déchargé du premier substrat (107) est plus grand que le nombre de chemins (108) d'introduction.
